# EUROPEAN PATENT APPLICATION

(11) **EP 2 145 642 A2**
(43) Date of publication of application: **20.01.2010**
(21) Application number: 09165506.8
(22) Date of filing: 15.07.2009
(51) Int. Cl.: A61M 5/42

(54) **Guiding and reminding device**

(30) Priority: 15.07.2008 HU 0800434
(71) Applicant: Kiss, Barnabás, 1037 Budapest (HU)
(72) Inventor: Kiss, Barnabás, 1037 Budapest (HU)
(74) Representative: Sipos, Jozsef

(57) **Abstract**

The application relates to a guiding and reminding device for determining the appropriate injection site. The device consists of at least two connected plates (1,5) that can be moved on each other and which are provided with holes (4,7) arranged in a certain system that can be brought in coincidence with each other as a needle insertion template, as well as with information carrier captions, drawings and signs. The novelty of the application is that one of the plates (1) is established as a date carrier plate with the consecutive numbers (2) corresponding to the days of a month along its edge part and with holes (4) arranged in a certain system which are assigned to the numbers (2) indicating the various days, while the other plate (5) of the device is established as a time carrier plate which is provided with numbers (6) corresponding to the various times, mostly the hours, or parts of a day. Site determination signs (3) are assigned to one of the date carrier plate (1) or the time carrier plate (5) for defining a more exact needle insertion point on the various body parts appointed for needle insertion, while the other plate is provided with symbolic signs (8) of the body parts suitable for needle insertion, and when the appropriate number (2) corresponding to the current date indicated on the date carrier plate (1) and the appropriate number (6) corresponding to the current time or part of a day indicated on the time carrier plate (5) are positioned next to each other, the optimal needle insertion point can be determined by reading the signs (3,8) assigned to the given date and time.

## Description

This invention relates to a guiding and reminding device for determining the appropriate injection site on various parts of the body and especially various parts of the skin surface, in particular for regularly performed medication injection and/or taking blood for testing. The device according to the invention recommends systematically injection and testing sites and parts of the body in order to prevent repeated needle insertions at the same site within a certain period of time.

When inserting the needle it is important to choose an area of the skin that has not been used for this purpose earlier. This is especially important when a needle is inserted regularly and repeatedly, e.g. for repeated medication injections, such as insulin injections by diabetics or in the case of repeated blood tests, tissue sampling or collecting of other body fluids. Hypodermic needles cannot be inserted into the same site for a while as not rotating injection sites may lead to fat formation on the skin, also known as lipodystrophy, which appears as a lump or swelling under the skin. The absorption of any medication injected into such a site becomes erratic: when the patient sometimes injects a needle into a healthy site and then into a lipo, absorption of the medication changes from occasion to occasion, thereby rendering its effect erratic. In the case of a diabetic who injects insulin regularly, use of the same site is particularly likely to cause lipodystrophy, resulting in changed levels of insulin absorption. This is dangerous or even fatal for the patient. Similarly to the foregoing, regular blood tests performed for checking blood sugar levels may also be unpleasant for patients, so it is recommended to rotate the needle insertion sites in such cases as well.

As a result of the above, it is desirable to rotate the site of regularly repeated injections so as to leave out previously used sites for a while. Several patents deal with the determination of the appropriate injection site of a hypodermic needle. For example, US Patent No. 3542022 shows a sheet divided into four parts, which helps to determine where the needle should be inserted, but only on a given body part. US Patent No. 2245350 offers an anesthetic injection guide using the anatomy, but only for the pelvic area, based on its size. US Patent No. 5634904 describes a template for knee injections. Canadian Patent No. 1140016 discloses an injection guide that uses a point of reference. US Patents No. 4228796 and 4362157, as well as the specification of W08000060 describe flexible perforated fabric sheets that can be used as a template for one daily injection. None of the solutions described in the above-cited patent documents is able to consider more than one body part for injections. In the case of these known solutions users must note down where a previous injection was administered. The user is only able to observe one body part with this method, which is one of the greatest deficiencies of these inventions. Using this method, if the user forgets to note the data or notes the data incorrectly, the injection may be administered at the same site. W002068028 suggests injection sites on various parts of the body based on the date, but only enables one injection per day, however, many patients require several daily injections, for which this device does not offer a simple solution. Several injections a day must be noted down or the body must be marked with the help of the device and subsequent injections have to be positioned in relation to this mark. US Patent No. 4349338 describes a daily injection site guide, which rotates the body parts recommended for medication injections daily, but does not show an exact position. Although the device described in US Patent No. 3999504 provides exact sites on various body parts, but is unable to suggest injection sites based on a day or time, so if the user forgets to mark the place or the mark is lost for some reason, the danger exists that the patient will use the same site again. The method requires a separate memo recorded on paper or an injection strategy with a marking plug to ensure that the user definitely finds a new injection site. At the same time, this device does not enable the user to search injection sites by date and time. In the case of the above-described inventions, recording of the injections and the use of a strategy or marking plug or mark requires extra effort and attention from the user, leading to potential errors and multiple injections into the same site.

W09302720 discloses an automatic programmable injection device which requires a complex and expensive background for administering injections with the use of a bar code. Similarly, the description of US Patent No. 5865744 includes a computerized system which determines the injection site based on images generated of the patient and the patient's anatomy. The device described in US Patent No. 6156008 detects the most suitable injection site, also taking into account the patient's blood pressure. These three patents, however, do not touch upon the problem of repeated injections, but do require significant and costly technical investment. The device described in W006061169 first assesses the skin's condition, then, after processing the data of the previous injection, points to a new place for administering the injection. The method requires a device to assess the skin's condition, as well as a device to process, analyze and categorize the data and a device to show the location of the injection. These factors render the above solution complicated and costly, so, similarly to the above three solutions, it can be used practically only in health institutions. In addition, the solution does not deal with saving the date and/or the time.

On the other hand, US Patent No. 6024723 discloses a relatively simple guiding device for performing blood tests from the sides of the fingers. This device consists of two disks that can be rotated in relation to each other around a central axis, with the drawing of a hand on each outside face and with two holes marking the sites where blood is drawn on each finger, under which, on the inside face of the other disk or a third disk placed in between, a prohibiting or allowing color field appears, thereby indicating to the user whether a given site is suitable for drawing blood. This device considers the side of the fingers suitable for drawing blood one after the other on both hands and needs to be rotated after each blood drawing. Another similar solution exists, which is also commercially available and consists of three disks that can be rotated in relation to each other and is suitable for determining the recommended consecutive insulin injections or blood testing sites. In order to achieve this, the outside face of one of the outside disks includes an image of the abdominal area with 20 potential injection sites, while the outside face of the other outside disk shows 20 potential blood drawing places on the fingers of the two hands, which needle insertion sites are numbered on both sides, and the numbers must be turned toward the corresponding arrow on the edge of the larger middle disk after each needle insertion (injection or blood drawing). Similarly to US Patent No. 6024723, hypodermic needle insertion sites must be determined consecutively, i.e. any one site can only be determined in relation to the previous injection site. At the same time, the device does not provide the injection or insertion site according to the day or the part of a day, so it does not allow searching for earlier sites if the number of blood tests or injections changes daily. If the user has forgotten to forward the disk or the disk does not show the previous insertion site for some other reason, the needle may subsequently be inserted at the same point on the skin. At the same time, due to their constructions, these devices cannot be used as a template.

The problem to be solved by the invention is to offer a simple, easy-to-manufacture and cheap guiding and reminding device that eliminates the deficiencies of the known solutions and which is able to determine the current optimal injection point from several possible body parts and sites or to determine the current optimal blood testing site from the suitable sites on the fingers of the hand based on the date and the time such that these injection and testing, i.e. needle insertion sites should not be the same within a certain period and by the use of which the user is able to search earlier injection and testing points based on a given date and time and which device can also be used as a needle insertion template.

This problem has been solved starting out from a device, which device consists of at least two connected plates that can be moved on each other and which are provided with holes arranged in a certain system that can be brought in coincidence with each other as a needle insertion template, as well as with information carrier captions, drawings and signs where the upper plate is smaller so that it only partially covers the plate underneath. According to the invention one of the plates of the device is established as a date carrier plate with the consecutive numbers of the days of the month along its edge part and with holes assigned to the days arranged in a certain system which are assigned to the numbers corresponding to a day, whereas the other plate of the device is established as a time carrier plate, with the different times - mostly the hours - of a day along its edge part. The time carrier plate is also provided with holes arranged according to a certain system, one of which is in coincidence with an appropriate hole of the date carrier plate when the appropriate number corresponding to the current date on the date carrier plate is placed next to the appropriate number corresponding to the current time or part of a day on the time carrier plate and thereby, through the coinciding holes, the user can see the optimal needle insertion point for that date and time. At the same time, site determination signs for defining a more exact site of the needle insertion point on the various body parts are assigned to one of the date carrier plate or the time carrier plate, while the other plate is provided with symbolic signs of the body parts suitable for needle insertion, and when the appropriate number on the date carrier plate and the appropriate number on the time carrier plate are positioned next to each other, the optimal needle insertion point can be determined by reading the signs assigned to the given date and time.

According to a particularly advantageous embodiment of the invention the larger plate of the device is established as the date carrier plate which is provided with the consecutive numbers of the days of a month along an uncovered edge part on its side not covered by the smaller time carrier plate, wherein directly next to the numbers site determination signs of the points on the body parts are arranged where the needle should be inserted and wherein the time carrier plate of the device is provided along its edge part with various times of the day, mostly the numbers of the hours, and symbolic signs of the optimal body parts for needle insertion are assigned to these numbers.

According to another possible version of the invention the plates are oblong and are clamped together with at least one strap allowing the two plates to slide in relation to each other.

With this version, the holes on the date carrier plate are arranged in several vertical columns, whereas the holes of the time carrier plate are arranged in groups the number of which is equal to the number of the vertical columns.

According to another advantageous embodiment of the invention the plates are round disks or polygonal two-dimensional figures which are clamped together along a central rotation axis and can be rotated in relation to each other.

With this embodiment the holes of the date carrier plate are evenly divided along several circles with differing radiuses and offset in relation to the holes on circles with other radiuses, while the holes of the time carrier plate are arranged one by one on the circles with differing radiuses in a number equal to the number of the various circles.

For the purposes of the invention, the device is especially advantageous in a form which not only provides the injection site but also the needle insertion point for blood tests. What is characteristic of such embodiment is that on the other side of the date carrier plate, which is not covered by the time carrier plate, there is a drawing of the fingers of a hand to which are assigned blood drawing points through connecting lines linked to the holes of the date carrier plate, in correlation with the setting of the current time and date of the time carrier plate and the date carrier plate on the other side of the date carrier plate by forming an appropriate coincidence of holes.

The symbolic signs of the body parts designated for needle insertion refer to areas suitable for injection on the abdomen, the upper thigh, the lower thigh and sometimes the arm, while the site determination signs for a more exact location on the body parts designated for needle insertion relate to the left/right or front/back side of the body part.

The aim to be achieved by the invention can be reached practically also with a simpler version of the device which consists of at least two connected plates that can be moved on each other and are provided with holes arranged in a certain system that can be brought in coincidence with each other and can be used as a needle insertion template and are supplied with data carrier captions, drawings and signs where the upper plate is of a different size than the lower plate. What is characteristic of this simplified form of the invention is that one of the plates of the device is established as a date carrier plate that is provided with the consecutive numbers of the days of the month one after the other in a circle. The other plate of the device is designed as a time carrier plate that is provided on its visible surface with a drawing of a body torso specifying all the body parts suitable for needle insertion, including signs related to the different body parts where the needle should be inserted, and assigned to them captions relating to the different parts of a day, wherein the numbers of the date carrier plate indicating the various days of a month appearing in a window type opening of the time carrier plate have assigned to them holes arranged in a certain system, while the date carrier plate is also provided with holes arranged in a certain system, one of which will be brought in coincidence upon the appearance of the number of the current date in the opening with an appropriate hole of the time carrier plate, thereby providing a needle insertion template with the optimal needle insertion point for that date and part of day where the device should be placed for needle insertion in accordance with the body torso.

According to a preferred embodiment for this simplified version of the device the plates are circular disks or polygonal two-dimensional figures that are clamped together along a central rotation axis and can be rotated in relation to each other, whereas the holes of the time carrier plate are evenly divided along several circles with differing radiuses and offset in relation to the holes on circles with other radiuses, while the holes on the date carrier plate are arranged one by one in a number equal to the number of the various circles.

It is advisable to create a version of this device that is suitable for determining the optimal blood testing site, where on the other side of the date carrier plate which is not covered by the time carrier plate there is a drawing of the fingers of at least one hand to which are assigned blood drawing points identifiable on the fingers arranged at the edge part of the time carrier plate, through connecting lines starting out from signs or captions referring to the various parts of a day, in correlation with the number of the current day that has been set in the opening of the time carrier plate.

The plates of the device are preferably rigid but they can be flexible to a certain extent. Accordingly, the material of the plates should be primarily carton or plastic but they can be made of other similar durable material that is at the same time pleasant for the touch.

The areas that can be used as injection sites depend on the medication, the age of the patient, how intact the area is and other factors. These potential areas must be divided in time. Suitable injection sites are e.g. the thigh, the abdomen or the arm. The right or left side of the body, as well as the upper or lower part of each body part, can be considered as separate areas for the purpose of injection sites. Accordingly the number of areas that can be used is 3x2x2, that is, 12. This means that these 12 areas must be divided according to date and hour (parts of a day). Other possible divisions exist, such as two areas on the thigh (upper and lower thigh) and the abdomen (altogether three variations), and within these, the right and left part of the body (two variations), and the front and side (two variations). Choosing one from each part results in altogether twelve different options for injection sites.

The invention will be understood from the following detailed description of preferred embodiments referring to the accompanying drawings.
*Fig. 1* shows a possible embodiment for the date carrier plate of an injection site guiding and reminding device consisting of rectangular plates,
*Fig. 2* shows a possible embodiment for the time carrier plate of an injection site guiding and reminding device consisting of rectangular plates.
*Fig. 3* shows an injection site guiding and reminding device consisting of a rectangular date carrier plate and a rectangular time carrier plate clamped together with a transparent strap,
*Fig. 4* shows a possible embodiment for the date carrier plate of an injection site guiding and reminding device consisting of disc-shaped plates,
*Fig. 5* shows a possible embodiment for the time carrier plate of an injection site guiding and reminding device consisting of disc-shaped plates,
*Fig. 6* shows a possible embodiment of the side suitable for determining blood test sites of an injection site guiding and reminding device consisting of disc-shaped plates,
*Fig. 7* shows a simplified embodiment of the injection site guiding and reminding device from the time carrier plate that can be applied as a template for determining needle insertion points, and
*Fig. 8* shows the side of the injection site guiding and reminding device according to Fig. 7 suitable for determining blood drawing points.

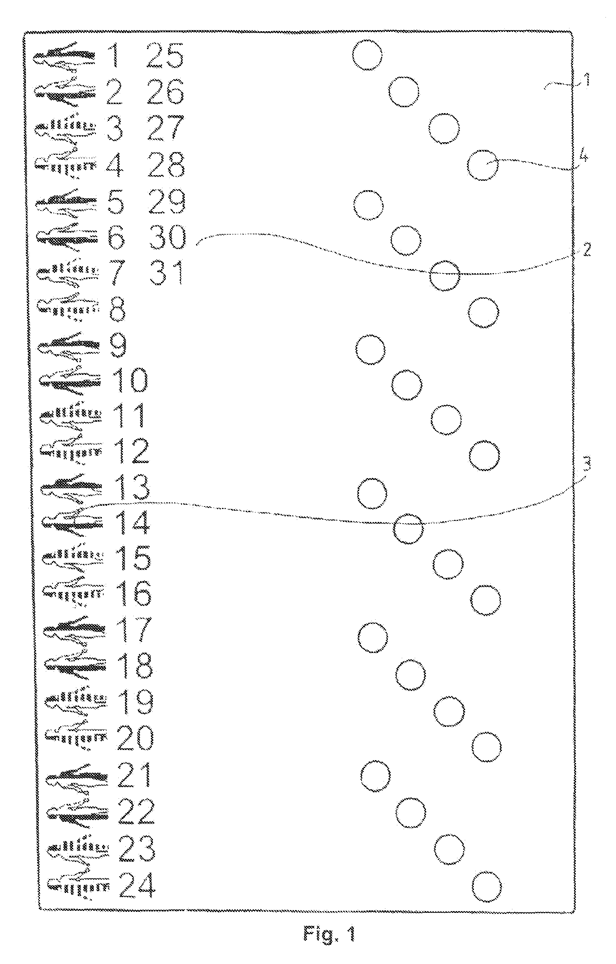

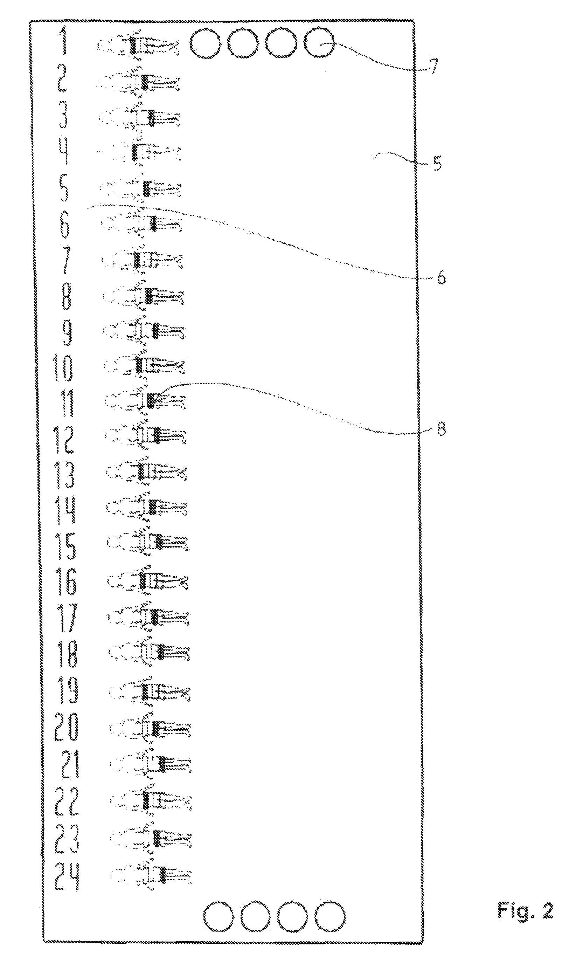

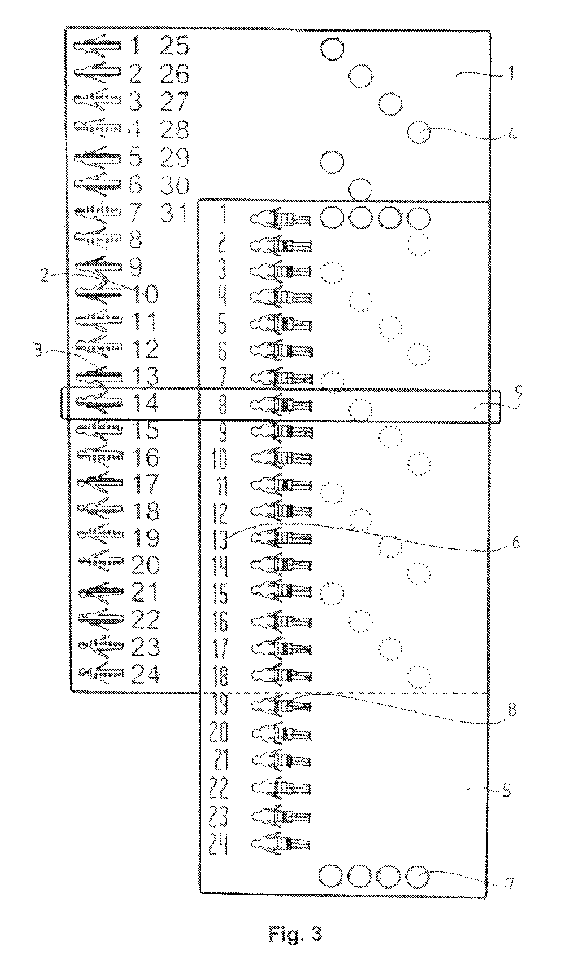

Figs. 1-3 show one possible embodiment of the injection site guiding and reminding device according to the invention which consists of rectangular plates 1 and 5 as shown in Figure 3. Instead of the rectangular shape, it is possible to design this version of the device in another oblong shape, eventually with rounded corners. The larger plate 1 indicated in Figure 1, which is partly covered by the plate 5, contains the dates, i.e. the consecutive numbers 2 of the days in a month along the left edge under each other, in some cases in two columns, so this plate will hereinafter be referred to as "date carrier plate 1". Site determination signs 3 are assigned to the consecutive numbers 2 indicating the date on the date carrier plate 1 which specify with appropriate symbols the exact injection site on a certain body part, i.e. the suitable side (right-left) or the front or back side of a given body part. In some cases the site determination signs 3 symbolize body forms, where the right or left side of the body designed for the injection is solid or striped, where the solid part means the front of the body part and the striped part means the side of the body part. On the date carrier plate 1, a hole 4 is assigned to each day, i.e. to each number 2, and said holes 4 are arranged in four vertical columns where the holes 4 belonging to the consecutive days always go to the next vertical column offset by a spacing in the vertical direction. In the assembled state of the device according to the invention as shown in Fig. 3, the plate 5 shown in Fig. 2 is arranged above the somewhat wider date carrier plate 1 shown in Fig. 1. The plate 5 is provided with the consecutive numbers 6 of the hours on the left edge to determine more accurately the time within each day, which will be referred to as "time carrier plate 5". Another feasible version of the device is where, instead of the hours of the day, the time carrier plate 5 is provided with time periods (sections of the day) or their symbols. When laid on top of each other, the plates 1 and 5, which cover each other partially, can be slid vertically in relation to each other, while the two plates 1 and 5 can be clamped together with e.g. a strap 9 as shown in Fig. 3. The strap should be of a transparent material and should be perpendicular to the direction of the sliding or sliding brims could be used. Symbolic signs 8 of the body parts where the needle should be inserted are assigned to the consecutive numbers 6 of the hours, and these symbolic signs, in the given case, relate to the surfaces of the abdomen, upper and lower thigh where the current area is marked in solid color. The arm is also a possible injection area. Additionally, the time carrier plate 5 has holes 7 arranged in a certain system, which are concordant with the holes 4 of the date carrier plate 1, in order to determine the most suitable injection site. When using the device, the user seeks the consecutive number 6 of the current hour on the time carrier plate 5 and slides it to the current date on the date carrier plate 1, i.e. to the consecutive number 2 indicating the given day. The user then reads the side of the body for the injection at an appropriate conjunction of the two plates 1 and 5 from the injection site determination sign 3 on the date carrier plate 1 and the exact position on the front or side of the body part in accordance with the symbol 8 on the time carrier plate 5. This will then be the exact surface of the body part where the injection has to be given. The exact injection site can be seen from the coinciding position of the holes 4 and 7 if the device is used as a template by being placed on the appropriate body part.

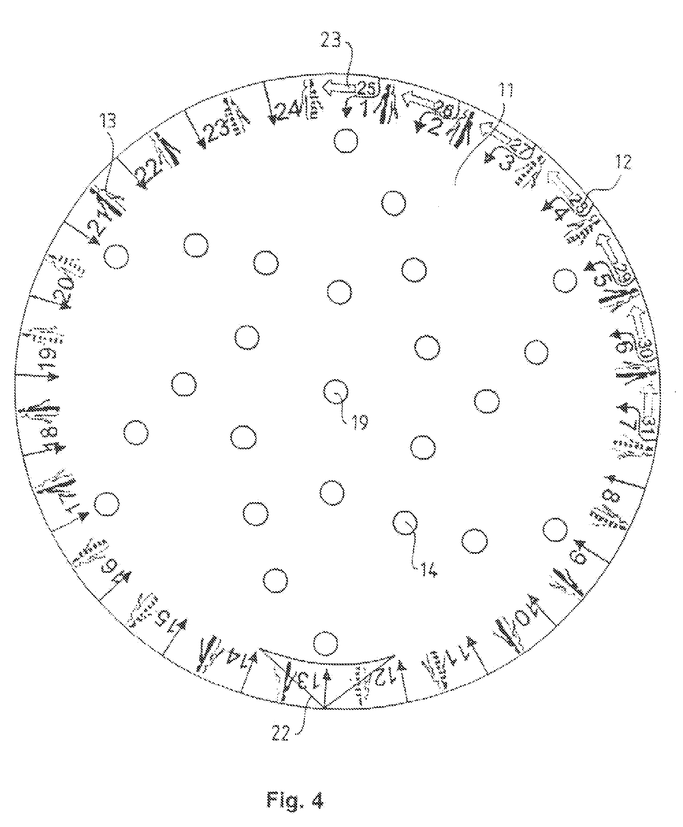

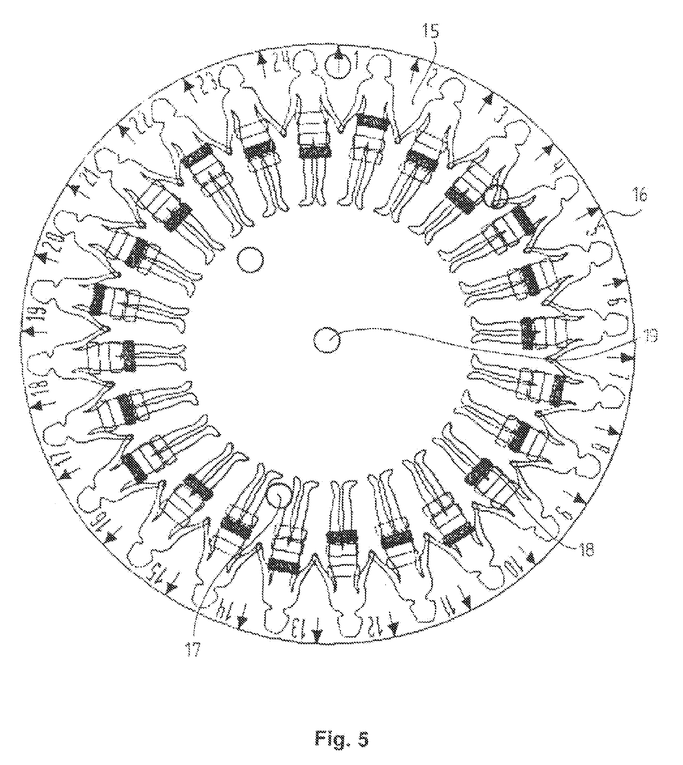

Figs. 4-6 show another possible embodiment of the injection site guiding and reminding device which consists of disk shaped plates 11 and 15, which are connected through a central axis 19 and can be slid in relation to each other. It is to be noted that instead of the circular disks as plates 11 and 15, we can use scalloped disks or plates of polygonal (hexagonal, octagonal, etc.) shapes. With this embodiment the rear date carrier plate 11 indicated in Fig. 4 includes the consecutive numbers 12 of the days in the month, and the site determination signs 13 which are assigned to the numbers 12 indicating the days, said site determination signs 13 specifying with symbols similar to the ones shown in Figs. 1-3 the injection site on a certain body part, i.e. the suitable side (right-left) or the front or back side of a given body part. On the date carrier plate 11, a hole 14 is assigned to each number 12 indicating a day, and said holes 14 are arranged in four circles with different radiuses, which circles are preferably coaxial, where said holes 14 belonging to the consecutive days always go to the next circle with a different radius. The time carrier plate 15, which has a somewhat smaller radius than the date carrier plate 11 beneath it, as seen in Fig. 5, contains the numbers 16 of the hours of the day (or in another possible embodiment, the time periods in one day) and the symbols 18 depicting the body parts assigned to the numbers 16, similarly to the numbers 6 and symbols 8 in Fig. 2. In addition, the time carrier plate 15 is provided with holes 17 cut in accordance with the holes 14 of the date carrier plate 11 to help determine the optimal injection site when applying the device as a template.

When using the above embodiment, the number 16 of the current hour is matched with the times (or parts of a day) indicated on the time carrier plate 15, which is then turned to the current date on the date carrier plate 11, i.e. to the number 12 indicating the given day. At this conjunction of the two disks 11 and 15 the user then reads from the signs 13 and 18 next to the number 12 of the current date and the number 16 of the current hour the appropriate side of the body indicated by the date carrier plate 11, and the appropriate front or side position within the indicated body part for the injection in accordance with the appropriate body part symbol shown on the time carrier plate 15. This will then be the exact surface of the body part where the injection has to be given. The exact injection site as a needle insertion template will be indicated by the coincidence of the holes 14 and 17 of the device laid on the indicated body part such that the date carrier plate 11 and the time carrier plate 15 rotated to each other according to the current date and time will be rotated together on the body part indicated so that the arrow 22 drawn between hour 12 and hour 13 on the date carrier plate 11 in Fig. 4 should point toward the user. The current hour must be rotated between the 25^{th} and 31^{st} day (of the current month) on the date carrier plate 11 in the same way under the current date as previously which shows the body part for the injection on the time carrier plate 15, however, as to the recommended body side and the front or side position within the body part, the site determination sign 13 for the previous day must be assigned to it on the date carrier plate 11, as indicated by the arrow 23 coupled to these dates. This embodiment allows different injection sites for at least 12 days. Depending on injection habits the average diabetic can achieve different injection sites for 31 consecutive days. If e.g. the time carrier plate 15 shows six symbols for time periods of a day instead of six hour-numbers, and the remaining hour segments are blank (generally diabetics need a maximum of six injections per day), using the device as described above, it allows a different injection site for 31 consecutive days.

In addition to determining the optimal injection sites, the above-described injection guide and reminder device can be made suitable for determining the optimal needle insertion points for blood tests.

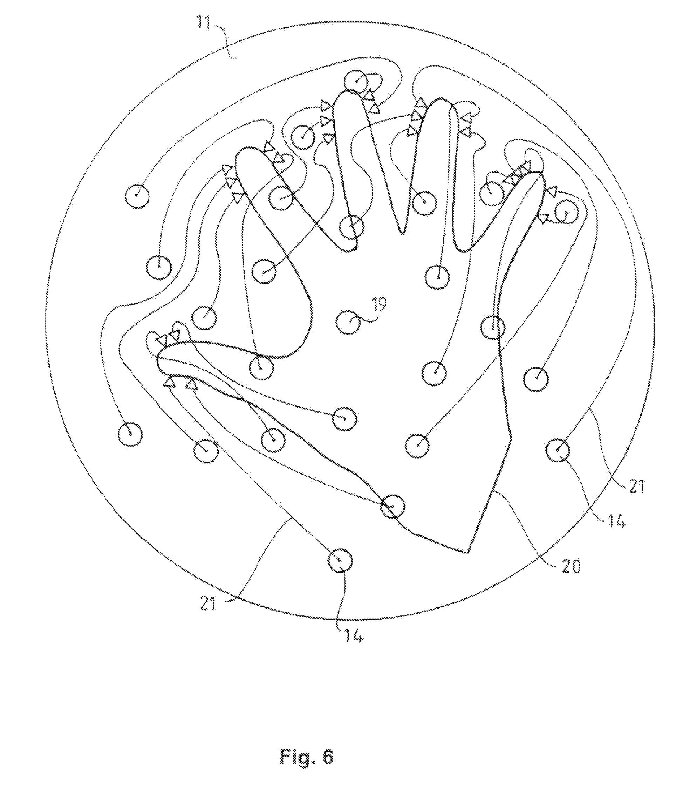

Fig. 6 shows a preferred embodiment of the injection guide and reminder device shown in Figs. 4 and 5. In the case of this embodiment, on the other side of the date carrier plate 11 not covered by the time carrier plate 15, the drawing of fingers 20 of a hand appears, to which are assigned with connecting lines 21 blood drawing points starting out from the holes 14 on the date carrier plate 11. As shown in Fig. 6, a possible solution is to assign four blood drawing points to the thumb and five to each of the other fingers. The position of coinciding holes 14 and 17 based on the date set on the injection side of the date carrier plate 11, i.e. the number 12 indicating the current day and the current hour 16 chosen on the time carrier plate 15 determines an injection site from where on the other side a connecting line 21 leads to a blood testing point on one of the fingers 20 of the hand. On the injection side of the date carrier plate 11 the suitable side (right or left) of the body, i.e. the right or left hand, is determined similarly to the injection site. In such case the device merely a guide and reminder but cannot be used as a template.

It should be noted that the embodiment according to Figs. 1-3 can also be made suitable for locating optimal testing and blood drawing sites as described above if a drawing of a hand is included on the other side of the date carrier plate 1 with connecting lines from the holes 4 to the blood drawing points. It should be also noted that the site determination signs 3; 13 which specify the location of the insertion are not necessarily placed on the date carrier plates 1; 11 but can be assigned to the time carrier plates 5; 15 after some adjustment, and the symbols 8; 18 for the body parts can be transferred to the date carrier plates 1; 11.

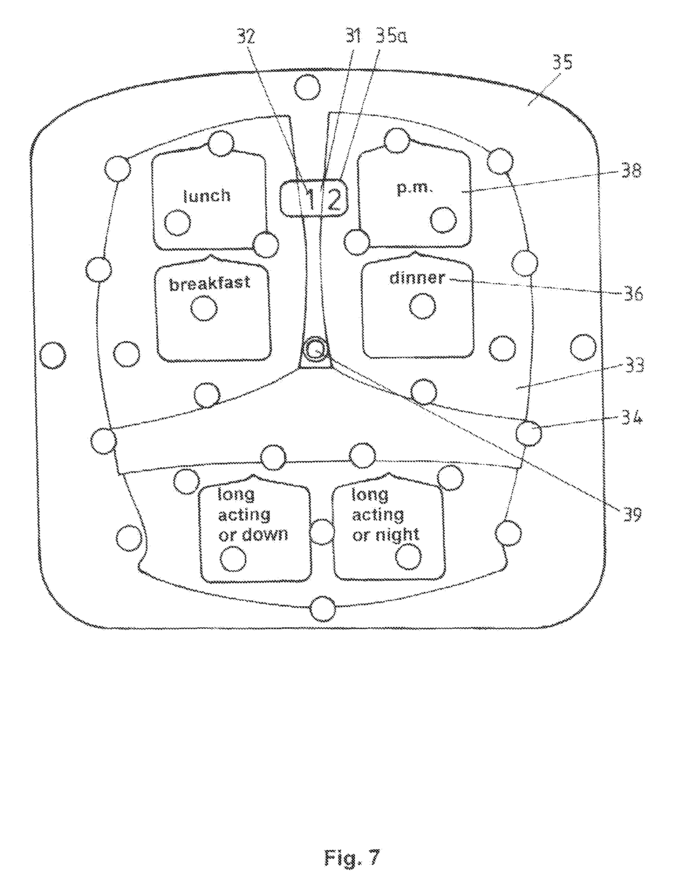

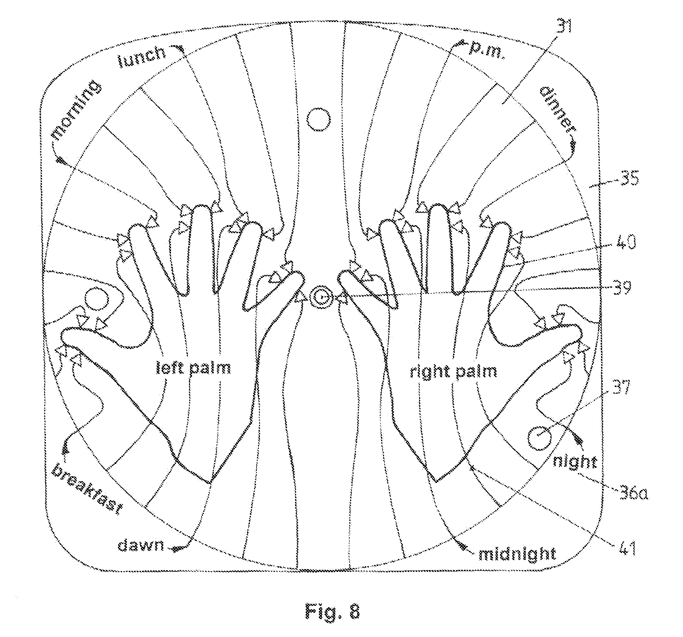

Figs. 7 and 8 show a simplified embodiment of the injection site guiding and reminding device which consists of a disk shaped date carrier plate 31 and a rounded rectangular time carrier plate 35, which are connected through a central axis 39 and can be slid in relation to each other. With this embodiment the other side of the date carrier plate 31 not visible on Fig. 8 contains the numbers 32 of the consecutive days of the month along a circle which can appear as shown in Fig. 7 in a window-type opening 35a of the time carrier plate 35 arranged above the date carrier plate 31. Fig. 7 also includes a drawing of a site determination body torso 33 on the visible surface of the time carrier plate 35 showing together all the body parts suitable for needle insertion, and showing the patient's body as he sees himself. The drawing of the body torso 33 includes signs 38 of the body parts where the needle should be inserted, and assigned to them signs or captions 36 with the different time periods of each day. Holes 34 are assigned according to a certain system to the numbers 32 corresponding to the consecutive days on the date carrier plate 31 and made visible in the window-like opening 35a of the time carrier plate 35. Said holes 34 are arranged in four coaxial circles with different radiuses where the holes 34 belonging to the consecutive days always go to the next circle with a different radius and are arranged in an offset way compared with the holes 34 on the other circles with different radiuses. At the same time on the date carrier plate 31 there are holes 37 arranged in accordance with the holes 34 of the time carrier plate 35 to determine the optimal injection site when using the device as a template.

When using the above-described simplified embodiment of the device, e.g. when injecting insulin, the user sets the current date by turning the date carrier plate 31 so that the current date is shown in the opening 35a of the time carrier plate 35. Thereafter, the user places the device on the body part indicated by the sign 38 in accordance with the chosen time period of a day (meal time, e.g. breakfast, etc.) as shown by the body torso 33 and the injection must be given where holes 34 and 37 coincide, so an opening is formed through the two plates.

The above-described simplified embodiment of the device can also be made suitable for locating optimal testing and blood drawing sites using the embodiment described in Fig. 8.

Under this embodiment of the device, on the other side of the date carrier plate 31 which is not covered by the time carrier plate 35, there is a drawing of the fingers 40 of two hands to which are assigned blood drawing points identifiable on the fingers arranged at the edge of the time carrier plate 35, through connecting lines 41 starting out from signs or captions 36a referring to various time periods of a day (or to corresponding meals). To determine blood testing and drawing points, the user sets the current date on the other side of the device in the opening 35a of the time carrier plate 35, then turns the device around and the appropriate connecting lines 41 lead to the recommended site on one of the fingers 40 of the hand from the sign or caption 36a referring to the current time period of a day. As seen, the coincidence of holes 34 and 37 plays no role in determining the testing and blood drawing site.

The device according to the invention is able to determine a body part to be injected and/or a needle injection point after a simple date and hour (or time of day) setting, which has not been injected in the recent days. The device is also able to determine for test injection, in particular for blood drawing such a blood drawing site after a simple date and hour (or time of day) setting, which has not been injected in the previous week. In both cases this new needle insertion point grants sufficient time for the previously injected points to heal. No notes must be taken of earlier injection sites, nor must the body or the device be marked. The device does not require a battery, nor does it require a screen or other data input device for finding the needle insertion point. In addition to being simple to handle, another advantage of the invention is that it is cheap to manufacture and practical to use.

## Claims

1. A guiding and reminding device for determining the appropriate injection site on various parts of the body, in particular for regularly performed medication injection and/or taking blood for testing, which device consists of at least two connected plates (1, 5; 11, 15) that can be moved on each other and which are provided with holes (4, 7; 14, 17) arranged in a certain system that can be brought in coincidence with each other as a needle insertion template as well as with information carrier captions, drawings and signs where the upper plate (5; 15) is smaller than the plate below (1; 11) so that it only partially covers the plate underneath, **characterized in that** one of the plates (1; 11) of the device is established as a date carrier plate with the consecutive numbers (2; 12) of the days of a month along its edge part and with holes (4; 14) arranged in a certain system which are assigned to the numbers (2; 12) corresponding to a day, whereas the other plate (5; 15) of the device is established as a time carrier plate which is provided along its edge part with numbers (6; 16) corresponding to the various times of each day, mostly the hours, or parts of a day, and this time carrier plate (5; 15) is also provided with holes (7, 17) arranged in a certain system, one of which is in coincidence with an appropriate hole (4; 14) of the date carrier plate (1; 11) when the appropriate number (2, 12) corresponding to the current date on the date carrier plate (1; 11) is placed next to the appropriate number (6; 16) corresponding to the current time or part of a day on the time carrier plate (5; 15), thereby giving through the coinciding holes a needle insertion template for the most suitable needle insertion point for that date and time, and wherein site determination signs (3; 13) are assigned to one of the date carrier plate (1; 11) and the time carrier plate (5; 15), for defining a more exact injection site on a body part appointed for needle insertion, while the other plate is provided with symbolic signs (8; 18) of the body parts suitable for needle insertion, and when the appropriate number (2; 12) corresponding to the current date indicated on the date carrier plate (1; 11) and the appropriate number (6; 16) corresponding to the current time or part of a day indicated on the time carrier plate (5; 15) are positioned next to each other, the optimal needle insertion point can be determined by reading the signs (3, 8; 13, 18) assigned to the given date and time.

2. A device according to claim 1, **characterized in that** the larger plate (1; 11) of the device is established as the date carrier plate which is provided with the consecutive numbers (2; 12) corresponding to the days of a month along an uncovered edge part on its side partly covered by the smaller time carrier plate (5; 15), wherein directly next to the numbers (2; 12) site determination signs (3; 13) are arranged for a more exact location of the optimal needle insertion point within the body part appointed for needle insertion, while the time carrier plate (5; 15) of the device is provided along its edge part with the various times of a day, mostly the numbers (6; 16) of the hours or parts of a day, and symbolic signs (8; 18) of the optimal body parts suitable for needle insertion are assigned thereto.

3. A device according to claim 1 or 2, **characterized in that** the plates (1; 5) are shaped as oblong, mostly rectangular plates and are clamped together with at least one strap (9) holding the two plates which can then slide in relation to each other, whereas the holes (4) on the date carrier plate (1) are arranged in several vertical columns, while the holes (7) of the time carrier plate (5) are arranged in groups the number of which is equal to the number of the vertical columns.

4. A device according to claim 1 or 2, **characterized in that** the plates (11; 15) are round disks or polygonal two-dimensional figures which are clamped together along a central rotation axis (19) and can be rotated in relation to each other, whereas the holes (14) of the date carrier plate (11) are evenly divided along several circles with differing radiuses and offset in relation to the holes on circles with other radiuses, while the holes (17) of the time carrier plate (15) are arranged one by one on the circles with differing radiuses in a number equal to the number of the various circles.

5. A device according to any of claims 1 to 4, **characterized in that** on the other side of date carrier plate (1; 11) not covered by the time carrier plate (5; 15), there is a drawing of the fingers (20) of a hand, to which are assigned blood drawing points on the fingers (20) through connecting lines (21) starting from the holes (4; 14) of the date carrier plate (1; 11), in correlation with setting the current time and date to each other on the date carrier plate (1; 11) and the time carrier plate (5, 15) on the other side of the date carrier plate (1; 11) and by forming an appropriate coincidence of holes.

6. A device according to any of claims 1 to 5, **characterized in that** the symbolic signs (8; 18) of the body parts appointed for needle insertion relate as areas suitable for injection to surfaces on the abdomen, the upper and lower thigh and sometimes the arm.

7. A device according to any of claims 1 to 6, **characterized in that** the site determination signs (3; 13) for a more exact location on the body parts appointed for needle insertion relate to the left/right or front/back side of the body parts

8. A guiding and reminding device for determining the appropriate injection site on various parts of the body, in particular for regularly performed medication injection and/or taking blood for testing, which device consists of at least two connected plates that can be moved on each other (31, 35) and are provided with holes (34, 37) arranged in a certain system that can be brought in coincidence with each other and can be used as a needle insertion template, as well as with information carrier captions, drawings and signs where the upper plate (35) is of a different size than the lower plate (31), **characterized in that** one of the plates of the device is established as a date carrier plate (31) that is provided with the consecutive numbers (32) corresponding to the days of a month one after the other along a circle, whereas the other plate of the device is established as a time carrier plate (35) that is provided on its visible surface with a drawing of a body torso (33) specifying all the body parts suitable for needle insertion, including signs (38) related to the different body parts where the needle should be inserted, and assigned to them captions (36) relating to the different parts of a day, wherein the numbers of the date carrier plate (31) indicating the various days of a month appearing in a window type opening (3 5 a) of the time carrier plate (35) have assigned to them holes (34) arranged in a certain system, while the date carrier plate (31) is also provided with holes arranged in a certain system, one of which will be brought in coincidence upon the appearance of the number of the current date in the opening (35a) with an appropriate hole of the time carrier plate (35), thereby providing a needle insertion template with the optimal needle insertion point for that date and part of day where the device should be placed for needle insertion in accordance with the body torso (33).

9. A device according to claim 8, **characterized in that** the plates (31, 35) are circular disks or polygonal two-dimensional figures that are clamped together along a central rotation axis (39) and can be rotated in relation to each other, whereas the holes (34) of the time carrier plate (35) are evenly divided along several circles with differing radiuses and offset in relation to the holes on circles with other radiuses, while the holes (31) on the time carrier plate (37) are arranged one by one in a number equal to the number of the various circles.

10. A device according to claim 8 or 9, **characterized in that** on the other side of the date carrier plate (31) which is not covered by the time carrier plate (35) there is a drawing of the fingers (40) of at least one hand to which are assigned blood drawing points identifiable on the fingers (40) arranged at the edge part of the time carrier plate (35), through connecting lines (41) starting out from signs or captions (36a) referring to the various parts of a day, in correlation with the number (32) of the current day on the date carrier plate (31) that has been set in the opening (35a) of the time carrier plate (35).
